(19) Europäisches Patentamt
European Patent Office
Office européen des brevets

(11) Numéro de publication : **0 035 619**
**B1**

(12) # FASCICULE DE BREVET EUROPÉEN

(45) Date de publication du fascicule du brevet : **25.07.84**

(51) Int. Cl.³ : **C 07 D239/96**, A 61 K 31/505

(21) Numéro de dépôt : **80400315.0**

(22) Date de dépôt : **10.03.80**

(54) **Dérivés d'amino-3 (1H,3H) quinazolinedione-2,4.**

(43) Date de publication de la demande :
**16.09.81 Bulletin 81/37**

(45) Mention de la délivrance du brevet :
**25.07.84 Bulletin 84/30**

(84) Etats contractants désignés :
**AT DE NL SE**

(56) Documents cités :
**EP-A- 0 012 051**
**AU-D- 612 466**
**JOURNAL OF ORGANIC CHEMISTRY, vol. 40, no. 13 (1975), pages 1909-1914 N.P. PEET, Sh. SUNDER: "Synthesis of 3,4-dihydro-1H-1,3,4-benzotriazepine-2,5-diones"**
**Le dossier contient des informations techniques présentées postérieurement au dépôt de la demande et ne figurant pas dans le présent fascicule.**

(73) Titulaire : **BERRI-BALZAC Société dite:**
**42, Rue Rouget-de-Lisle**
**F-92151 Suresnes Cedex (FR)**

(72) Inventeur : **Baronnet, René**
**15 Bis, Rue Henri Regnault**
**F-92300 Garches (FR)**
Inventeur : **Callendret, Raymond**
**1 Bis, Rue Bellavoine**
**F-78230 Le Pecq (FR)**

(74) Mandataire : **Bressand, Georges et al**
**c/o CABINET LAVOIX 2 Place d'Estienne d'Orves**
**F-75441 Paris Cedex 09 (FR)**

**EP 0 035 619 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description** (pour les Etats contractants : DE, NL, SE)

La présente invention a pour objet un médicament à activité anxiolytique, diurétique et spasmolytique contenant, à titre de principe actif, un dérivé d'amino-3 (1H, 3H) quinazolinedione-2,4 répondant à la formule :

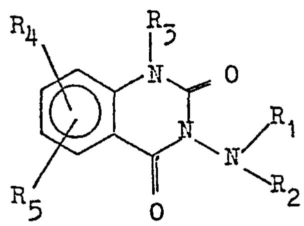

(I)

dans laquelle $R_1$ et $R_2$ représentent chacun, indépendamment l'un de l'autre, un atome d'hydrogène, un groupe alkyle en $C_1$ à $C_4$ ou un groupe acétyle, $R_1$ et $R_2$ n'étant pas simultanément l'hydrogène ; $R_3$ représente un groupe alkyle en $C_1$ à $C_4$ ; et $R_4$ et $R_5$ représentent chacun un atome d'hydrogène ou d'halogène, un groupe alkyle en $C_1$ à $C_4$ ou un groupe alcoxy en $C_1$ à $C_4$.

L'invention comprend aussi l'utilisation comme principe actif des sels d'addition des composés ci-dessus avec des acides (notamment l'acide chlorhydrique, l'acide bromhydrique et l'acide sulfurique).

Ces composés possèdent une action anxiolytique accompagnée d'une action diurétique et d'une action spasmolytique de nature non α-adrénalitique sur les fibres musculaires lisses, activités qui en font des substances utiles dans le traitement de l'hypertension.

On connaît déjà par le brevet australien AU-B-6124/66 des composés de formule générale

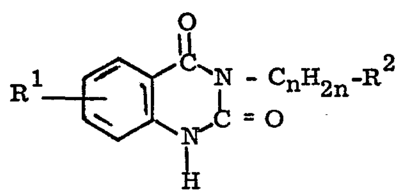

qui, lorsque n = 0, sont analogues aux composés de la présente invention, une des différences essentielles résidant dans le fait que, dans les composés du brevet australien, l'autre azote cyclique porte un atome d'hydrogène alors que dans la présente invention les composés portent un groupe alkyle en $C_1$ à $C_4$. Les composés du brevet australien sont indiqués comme ayant une activité sédative et anti-inflammatoire.

Cependant, ces composés ne présentent pas une activité sédative combinée à une diurèse suffisante pour pouvoir être valablement utilisés dans le traitement de l'hypertension comme c'est le cas avec les composés de formule (I) utilisés comme principe actif dans le médicament de la présente invention.

Rien dans le document précité ne suggère au spécialiste d'utiliser plus particulièrement des dérivés dans lesquels n = 0 et de remplacer dans ceux-ci l'atome d'hydrogène sur l'azote cyclique par un groupe alkyle inférieur.

La demande de brevet européen EP-A-12051 décrit, à titre de produits intermédiaires, pour l'obtention de dérivés thérapeutiquement actifs, certains dérivés entrant dans le cadre de la formule générale (I) précitée, mais ce document ne décrit ni ne suggère des propriétés pharmacologiques ou thérapeutiques desdits dérivés.

Pour préparer les dérivés de formule (I), on fait réagir un acide anthranilique de formule :

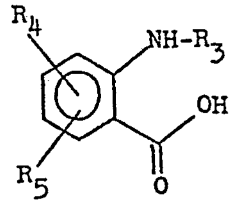

(II)

dans laquelle $R_3$, $R_4$ et $R_5$ ont les définitions précitées, avec du phosgène, obtenant ainsi un anhydride isatoïque de formule :

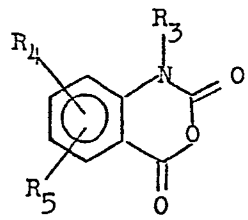

(III)

que l'on fait réagir ensuite sur une hydrazine de formule :

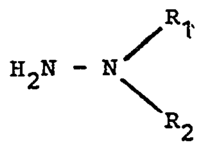

(IV)

dans laquelle $R_1$ et $R_2$ ont les significations précitées, obtenant ainsi un hydrazide anthranilique de formule :

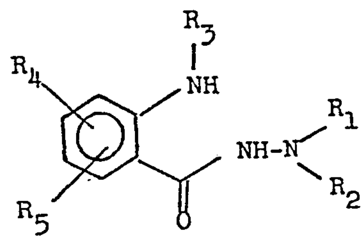

(V)

que l'on cyclise ensuite par réaction avec du phosgène ou du chloroformiate d'éthyle pour obtenir le composé de formule (I).

Ce procédé peut être schématisé comme suit :

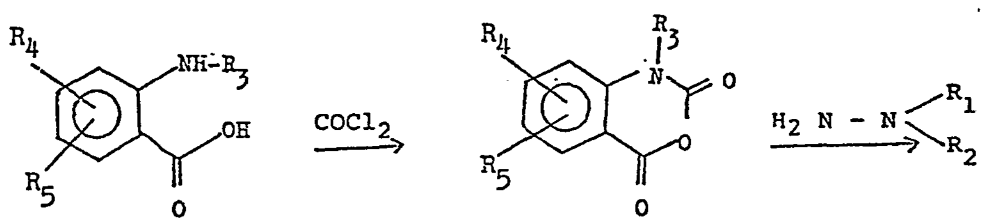


(II) (III) (IV)

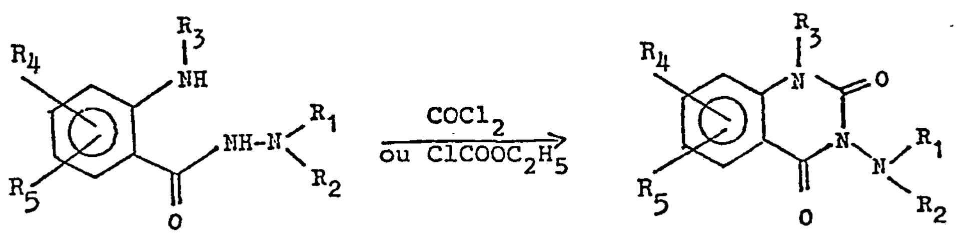


(V) (I)

Suivant une variante, pour obtenir les composés dans lesquels $R_1$ et $R_2$ représentent tous deux un groupe acétyle, on part du composé correspondant dans lequel l'un des symboles $R_1$ et $R_2$ est un groupe acétyle et on le fait réagir avec un agent d'acétylation pour introduire le second groupe acétyle.

On met le procédé en œuvre, notamment de la façon suivante :

Procédé général de fabrication

a) Préparation de l'anhydride isatoïque de formule (III) :

L'acide anthranilique (II) convenablement substitué est dissous dans une solution aqueuse d'acide chlorhydrique ou éventuellement dans le dioxane lorsque cet acide n'est pas soluble en milieu acide. On fait passer dans cette solution un courant de chlorure de carbonyle (phosgène). L'anhydride isatoïque (III)

précipite peu à peu. Après 1 h 30 de contact, le produit est essoré, rincé à l'eau et séché à l'étuve à 100 °C. Les eaux-mères peuvent subir l'action d'un deuxième contact avec le phosgène pour fournir un autre jet de cristallisation.

b) Préparation de l'hydrazide anthranilique de formule (V) :

A une suspension dans un alcool aliphatique, ou mieux dans un éther alcoylique de l'éthylène glycol, notamment l'éther diméthylique, de 1 mole d'anhydride isatoïque (III), on ajoute 1 mole d'hydrazine (IV). On laisse le mélange en réaction jusqu'à cessation de dégagement d'anhydride carbonique. Le solvant est partiellement éliminé sous pression réduite et le produit de réaction est généralement cristallisé par addition de benzène. La pureté du produit est suffisante pour l'étape ultérieure.

c) Cyclisation des hydrazides anthraniliques (V) en (1H, 3H) quinazolinediones-2,4 de formule (I)

Méthode A :

On dissout dans le minimum d'acide chlorhydrique 2N l'hydrazide (V) précédemment obtenu. La solution est alors traitée au chlorure de carbonyle. Le produit de réaction précipite lentement dans le milieu. Après 1 h de contact, le produit est essoré, rincé à l'eau et séché. Il est purifié par recristallisation dans l'éthanol ou l'isopropanol.

Méthode B :

L'hydrazide (V) est cyclisé en amino-3 (1H, 3H) quinazolinedione-2,4 en chauffant sous reflux le produit en présence de chloroformiate d'éthyle pendant 3 à 4 h. L'excès de chloroformiate d'éthyle est éliminé par distillation et le résidu est cristallisé à partir de l'éthanol.

Les exemples non limitatifs suivants illustrent le procédé de l'invention.

### Exemple 1

Préparation de l'anhydride diméthoxy-6,7 isatoïque

49,5 g d'acide amino-2 diméthoxy-4,5 benzoïque sont dissous dans 1 litre d'acide chlorhydrique 2N. On fait passer à travers le mélange agité un courant de phosgène pendant 1 h 30 en maintenant la température inférieure à 40°. Le milieu réactionnel est purgé au moyen d'un courant d'air et le précipité obtenu est filtré, lavé à l'eau et séché à l'étuve à 100 °C. F = 291 °C.

### Exemple 2

Préparation de l'amino-2N-morpholinobenzamide

33 g d'anhydride isatoïque sont placés en suspension dans 200 $cm^3$ d'éthanol porté à 70 °C ; on ajoute lentement 20,5 g d'amino-4 morpholine. Après addition, la température est maintenue jusqu'à cessation de dégagement d'anhydride carbonique. L'éthanol est partiellement éliminé (100 $cm^3$) et le produit de réaction est cristallisé par addition de 100 $cm^3$ de benzène. Il peut être purifié par recristallisation dans un mélange benzène-éthanol. F = 172 °C.

### Exemple 3

Préparation de la diméthylamino-3 méthyl-1 (1H, 3H) quinazolinedione-2,4 (cyclisation au chloroformiate d'éthyle)

19,3 g de méthylamino-2 N-diméthylaminobenzamide sont chauffés sous reflux dans 100 $cm^3$ de chloroformiate d'éthyle pendant 4 h. L'excès de chloroformiate d'éthyle est éliminé par distillation et le résidu est cristallisé à partir de l'éthanol. F = 154 °C.

### Exemple 4

Préparation de la diacétylamino-3 (1H, 3H) quinazolinedione-2,4

4 g de N-acétylamino-3 (1H, 3H) quinazolinedione-2,4 sont chauffés sous reflux dans 20 $cm^3$ d'anhydride acétique pendant 5 h. Après quoi, l'excès d'anhydride acétique est éliminé par distillation sous pression réduite. Le résidu obtenu est cristallisé à partir du benzène. F = 210 °C.

De la même manière on a préparé les composés ci-dessous de formule (I).

| | Point de fusion |
|---|---|
| diéthylamino-3 méthyl-1 (1H, 3H) quinazolinedione-2,4 | 178 °C |
| diméthylamino-3 éthyl-1 (1H, 3H) quinazolinedione-2,4 | 98 °C |
| chloro-7 diméthylamino-3 éthyl-1 (1H, 3H) quinazolinedione-2,4 | 143 °C |
| chloro-7 diméthylamino-3 isopropyl-1 (1H, 3H) quinazolinedione-2,4 | 100 °C |

Le principe actif du médicament de l'invention est généralement associé à un véhicule pharmaceutiquement administrable approprié.

Les résultats d'essais toxicologiques et pharmacologiques rapportés ci-dessous illustrent les activités des composés de formule (I).

A. Toxicité

Les toxicités aiguës ont été déterminées sur la souris Swiss.

Par exemple, pour le composé de l'exemple 3, la $DL_{50}$ par voie IP est de 250 mg et per os de 225 mg.

B. Action diurétique

L'action diurétique est mesurée sur le rat vigile traité par 25 ou 50 mg/kg de substance à tester prise par voie orale ou intrapéritonéale et recevant une injection I.P. de soluté isotonique de chlorure de sodium au début de l'essai. Les rats sont groupés par trois et les expériences sont répétées en croisant les lots de rats.

La diurèse est exprimée par le pourcentage de l'augmentation moyenne de la diurèse par rapport à la moyenne des témoins.

Les résultats pour le composé de l'exemple 3 sont donnés dans le Tableau I ci-dessous :

Tableau I

| | Diurèse | | | |
|---|---|---|---|---|
| | Dose en mg/kg | | | |
| | par voie IP | | par voie orale | |
| | 25 | 50 | 25 | 50 |
| Ex. 3 | 60 | 200 | 77 | 200 |

C. Action sédative

L'action sédative est mesurée par un indice de sédation. Cet indice de sédation tient compte du pourcentage, chez la souris, de la diminution de l'exploration de la planche à trous par rapport à des témoins, de l'hypothermie, de la mobilité, de l'agilité. Les résultats pour le composé de l'exemple 3, sont donnés au Tableau II.

Tableau II

| Indice de sédation | | | Indice de sédation chimique |
|---|---|---|---|
| 100 mg/kg voie I.P. | 50 mg/kg per os | 100 mg/kg per os | 100 mg/kg per os |
| 236 | 76 | 164 | 179 |

D'autre part, il faut noter une potentialisation par les composés de formule (I) des psychotropes : Nembutal, Chlorpromazine, morphine, et un antagoniste de l'amphétamine. A titre d'exemple, le composé de l'exemple 3 potentialise l'effet hypnotique du Nembutal de + 146 %, celui de la Chlorpromazine de + 50 % et celui de la morphine de 40 %. Par contre, il antagonise à 70 % les effets de l'amphétamine.

Les composés de formule (I) ont donc des propriétés sédatives sur le système nerveux central du type « anxiolytique ».

Ils sont particulièrement utilisables pour le traitement de l'hypertension.

Les composés de formule (I) sont notamment administrables par voie orale et rectale.

Pour ces modes d'administration, le médicament est avantageusement formulé en doses unitaires telles que comprimés, gélules et suppositoires, dans lesquelles le principe actif est associé aux excipients et véhicules pharmaceutiques usuels et appropriés.

Chaque dose unitaire contient avantageusement de 5 mg à 30 mg de principe actif.

On donnera ci-dessous, à titre illustratif, une formulation de comprimé :

| | |
|---|---|
| Diméthylamino-3 méthyl-1 (1H, 3H) quinazolinedione-2,4 | 10 mg |
| excipient q.s.p. un comprimé de | 150 mg |

**Description** (pour l'Etat contractant AT)

La présente invention a pour objet un procédé de préparation de nouveaux dérivés d'amino-3 (1H, 3H) quinazolinedione-2,4 utilisables en thérapeutique, notamment comme anxiolytiques, diurétiques et spasmolytiques.

Les nouveaux composés répondent à la formule :

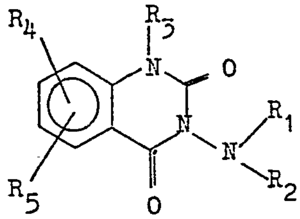

(I)

dans laquelle $R_1$ et $R_2$ représentent chacun, indépendamment l'un de l'autre, un atome d'hydrogène, un groupe alkyle en $C_1$ à $C_4$ ou un groupe acétyle $R_1$ et $R_2$ n'étant pas simultanément l'hydrogène ; $R_3$ représente un groupe alkyle en $C_1$ à $C_4$ ; et $R_4$ et $R_5$ représentent chacun un atome d'hydrogène ou d'halogène, un groupe alkyle en $C_1$ à $C_4$ ou un groupe alcoxy en $C_1$ à $C_4$.

L'invention comprend aussi la préparation des sels d'addition des composés ci-dessus avec des acides (notamment l'acide chlorhydrique, l'acide bromhydrique et l'acide sulfurique).

Ces nouveaux composés possèdent une action anxiolytique accompagnée d'une action diurétique et d'une action spasmolytique de nature non λ-adrénalitique sur les fibres musculaires lisses, activités qui en font des substances utiles dans le traitement de l'hypertension. Ils peuvent en outre servir d'intermédiaires de synthèse.

On connaît déjà par le brevet australien AU-B-6124/66 des composés de formule générale

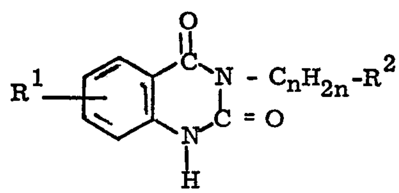

qui, lorsque n = 0, sont analogues aux composés de la présente invention, une des différences essentielles résidant dans le fait que, dans les composés du brevet australien, l'autre azote cyclique porte un atome d'hydrogène alors que dans la présente invention les composés portent un groupe alkyle en $C_1$ à $C_4$. Les composés du brevet australien sont indiqués comme ayant une activité sédative et anti-inflammatoire.

Cependant, ces composés ne présentent pas une activité sédative combinée à une diurèse suffisante pour pouvoir être valablement utilisés dans le traitement de l'hypertension comme c'est le cas avec les composés de formule (I) utilisés comme principe actif dans le médicament de la présente invention.

Rien dans le document précité ne suggère au spécialiste d'utiliser plus particulièrement des dérivés dans lesquels n = 0 et de remplacer dans ceux-ci l'atome d'hydrogène sur l'azote cyclique par un groupe alkyle inférieur.

L'invention a donc pour objet un procédé de préparation des composés de formule (I) précitée, caractérisé en ce qu'on fait réagir un acide anthranilique de formule :

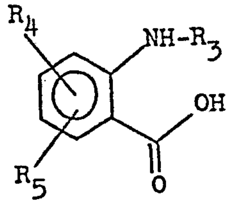

(II)

dans laquelle $R_3$, $R_4$ et $R_5$ ont les définitions précitées, avec du phosgène, obtenant ainsi un anhydride isatoïque de formule :

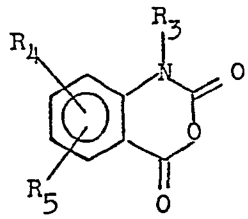

(III)

que l'on fait réagir ensuite sur une hydrazine de formule :

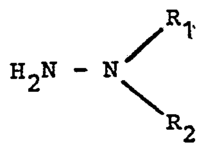

(IV)

dans laquelle $R_1$ et $R_2$ ont les significations précitées, obtenant ainsi un hydrazide anthranilique de formule :

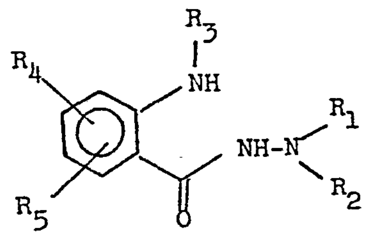

(V)

que l'on cyclise ensuite par réaction avec du phosgène ou du chloroformiate d'éthyle pour obtenir le composé de formule (I).

Le procédé de l'invention peut être schématisé comme suit :

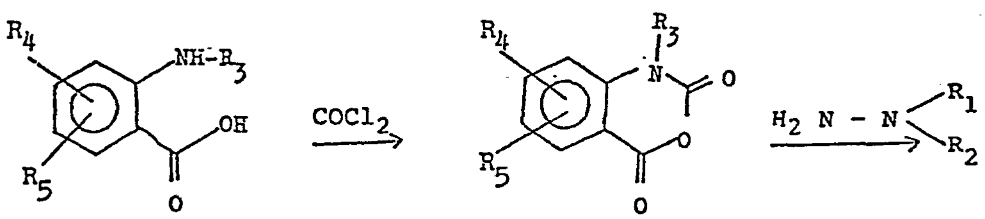


(II) (III) (IV)

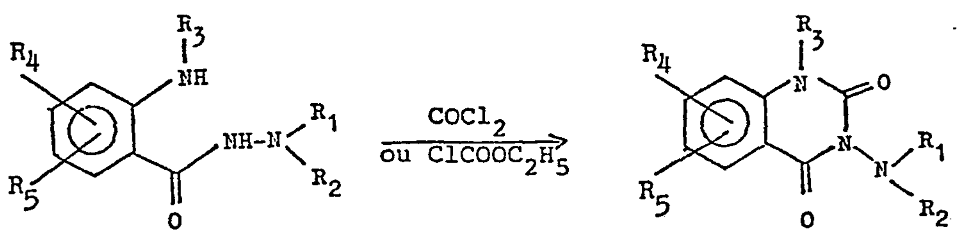


(V) (I)

Suivant une variante, pour obtenir les composés dans lesquels $R_1$ et $R_2$ représentent tous deux un groupe acétyle, on part du composé correspondant dans lequel l'un des symboles $R_1$ et $R_2$ est un groupe acétyle et on le fait réagir avec un agent d'acétylation pour introduire le second groupe acétyle.

On met le procédé en œuvre, notamment de la façon suivante :

Procédé général de fabrication

a) Préparation de l'anhydride isatoïque de formule (III)

L'acide anthranilique (II) convenablement substitué est dissous dans une solution aqueuse d'acide chlorhydrique ou éventuellement dans le dioxane lorsque cet acide n'est pas soluble en milieu acide. On fait passer dans cette solution un courant de chlorure de carbonyle (phosgène). L'anhydride isatoïque (III) précipite peu à peu. Après 1 h 30 de contact, le produit est essoré, rincé à l'eau et séché à l'étuve à 100 °C. Les eaux-mères peuvent subir l'action d'un deuxième contact avec le phosgène pour fournir un autre jet de cristallisation.

b) Préparation de l'hydrazide anthranilique de formule (V)

A une suspension dans un alcool aliphatique, ou mieux dans un éther alcoylique de l'éthylène glycol, notamment l'éther diméthylique, de 1 mole d'anhydride isatoïque (III), on ajoute 1 mole d'hydrazine (IV). On laisse le mélange en réaction jusqu'à cessation de dégagement d'anhydride carbonique. Le solvant est partiellement éliminé sous pression réduite et le produit de réaction est généralement cristallisé par addition de benzène. La pureté du produit est suffisante pour l'étape ultérieure.

c) Cyclisation des hydrazides anthraniliques (V) en (1H, 3H) quinazolinediones-2,4 de formule (I)

Méthode A :

On dissout dans le minimum d'acide chlorhydrique 2N l'hydrazide (V) précédemment obtenu. La solution est alors traitée au chlorure de carbonyle. Le produit de réaction précipite lentement dans le milieu. Après 1 h de contact, le produit est essoré, rincé à l'eau et séché. Il est purifié par recristallisation dans l'éthanol ou l'isopropanol.

Méthode B :

L'hydrazide (V) est cyclisé en amino-3 (1H, 3H) quinazolinedione-2,4 en chauffant sous reflux le produit en présence de chloroformiate d'éthyle est éliminé par distillation et le résidu est cristallisé à partir de l'éthanol.

Les exemples non limitatifs suivants illustrent le procédé de l'invention.

Exemple 1

Préparation de l'anhydride diméthoxy-6,7 isatoïque

49,5 g d'acide amino-2 diméthoxy-4,5 benzoïque sont dissous dans 1 litre d'acide chlorhydrique 2N. On fait passer à travers le mélange agité un courant de phosgène pendant 1 h 30 en maintenant la température inférieure à 40°. Le milieu réactionnel est purgé au moyen d'un courant d'air et le précipité obtenu est filtré, lavé à l'eau et séché à l'étude à 100 °C. F = 291 °C.

Exemple 2

Préparation de l'amino-2N-morpholinobenzamide

33 g d'anhydride isatoïque sont placés en suspension dans 200 $cm^3$ d'éthanol porté à 70 °C ; on ajoute lentement 20,5 g d'amino-4 morpholine. Après addition, la température est maintenue jusqu'à cessation de dégagement d'anhydride carbonique. L'éthanol est partiellement éliminé (100 $cm^3$) et le produit de réaction est cristallisé par addition de 100 $cm^3$ de benzène. Il peut être purifié par recristallisation dans un mélange benzène-éthanol. F = 172 °C.

Exemple 3

Préparation de la diméthylamino-3 méthyl-1(1H, 3H) quinazolinedione-2,4 (cyclisation au chloroformiate d'éthyle)

19,3 g de méthylamino-2 N-diméthylaminobenzamide sont chauffés sous reflux dans 100 $cm^3$ de chloroformiate d'éthyle pendant 4 h. L'excès de chloroformiate d'éthyle est éliminé par distillation et le résidu est cristallisé à partir de l'éthanol. F = 154 °C.

Exemple 4

Préparation de la diacétylamino-3 (1H, 3H) quinazolinedione-2,4

4 g de N-acétylamino-3 (1H, 3H) quinazolinedione-2,4 sont chauffés sous reflux dans 20 $cm^3$

d'anhydride acétique pendant 5 h. Après quoi, l'excès d'anhydride acétique est éliminé par distillation sous pression réduite. Le résidu obtenu est cristallisé à partir du benzène. F = 210 °C.

De la même manière on a préparé les composés ci-dessous de formule (I).

| | Point de fusion |
|---|---|
| diéthylamino-3 méthyl-1(1H, 3H) quinazolinedione-2,4 | 178 °C |
| diméthylamino-3 éthyl-1(1H, 3H) quinazolinedione-2,4 | 98 °C |
| chloro-7 diméthylamino-3 éthyl-1(1H, 3H) quinazolinedione-2,4 | 143 °C |
| chloro-7 diméthylamino-3 isopropyl-1(1H, 3H) quinazolinedione-2,4 | 100 °C |

Les résultats d'essais toxicologiques et pharmacologiques rapportés ci-dessous illustrent les activités des composés de formule (I).

A. Toxicité

Les toxicités aiguës ont été déterminées sur la souris Swiss.

Par exemple, pour le composé de l'exemple 3, la $DL_{50}$ par voie IP est de 250 mg et per os de 225 mg.

B. Action diurétique

L'action diurétique est mesurée sur le rat vigile traité par 25 ou 50 mg/kg de substance à tester prise par voie orale ou intrapéritonéale et recevant une injection IP de soluté isotonique de chlorure de sodium au début de l'essai. Les rats sont groupés par trois et les expériences sont répétées en croisant les lots de rats.

La diurèse est exprimée par le pourcentage de l'augmentation moyenne de la diurèse par rapport à la moyenne des témoins.

Les résultats pour le composé de l'exemple 3 sont donnés dans le Tableau I ci-dessous :

Tableau I

| | Diurèse | | | |
|---|---|---|---|---|
| | Dose en mg/kg | | | |
| | par voie IP | | par voie orale | |
| | 25 | 50 | 25 | 50 |
| Ex. 3 | 60 | 200 | 77 | 200 |

C. Action sédative

L'action sédative est mesurée par un indice de sédation. Cet indice de sédation tient compte du pourcentage, chez la souris, de la diminution de l'exploration de la planche à trous par rapport à des témoins, de l'hypothermie, de la mobilité, de l'agilité. Les résultats pour le composé de l'exemple 3 sont donnés au Tableau II.

Tableau II

| Indice de sédation | | | Indice de sédation chimique |
|---|---|---|---|
| 100 mg/kg voie I.P. | 50 mg/kg per os | 100 mg/kg per os | 100 mg/kg per os |
| 236 | 76 | 164 | 179 |

D'autre part, il faut noter une potentialisation par les composés de formule (I) des psychotropes : Nembutal, Chlorpromazine, morphine et un antagoniste de l'amphétamine. A titre d'exemple, le composé de l'exemple 3 potentialise l'effet hypnotique du Nembutal de + 146 %, celui de la Chlorpromazine de + 50 % et celui de la morphine de 40 %. Par contre, il antagonise à 70 % les effets de l'amphétamine.

Les composés de formule (I) ont donc des propriétés sédatives sur le système nerveux central du type « anxiolytique ».

Ils sont particulièrement utilisables pour le traitement de l'hypertension.

Les composés de formule (I) sont notamment administrables par voie orale et rectale.

Pour ces modes d'administration, le médicament est avantageusement formulé en doses unitaires telles que comprimés, gélules et suppositoires, dans lesquelles le principe actif est associé aux excipients et véhicules pharmaceutiques usuels et appropriés.

Chaque dose unitaire contient avantageusement de 5 mg à 30 mg de principe actif.

On donnera ci-dessous, à titre illustratif, une formulation de comprimé :

| | |
|---|---|
| Diméthylamino-3 méthyl-1(1H, 3H) quinazolinedione-2,4 | 10 mg |
| excipient q.s.p. un comprimé de | 150 mg. |

**Revendications** (pour les Etats contractants : DE, NL, SE)

1. Médicament à activité anxiolytique, diurétique et spasmolytique utilisable pour le traitement de l'hypertension, caractérisé en ce qu'il contient, à titre de principe actif, un composé de formule :

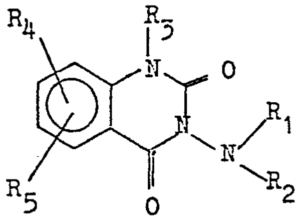

(I)

dans laquelle $R_1$ et $R_2$ représentent chacun, indépendamment l'un de l'autre, un atome d'hydrogène, un groupe alkyle en $C_1$ à $C_4$ ou un groupe acétyle, $R_1$ et $R_2$ n'étant pas simultanément l'hydrogène ; $R_3$ représente un groupe alkyle en $C_1$ à $C_4$ ; et $R_4$ et $R_5$ représentent chacun un atome d'hydrogène ou d'halogène, un groupe alkyle en $C_1$ à $C_4$ ou un groupe alcoxy en $C_1$ à $C_4$ ; ou un sel d'addition avec un acide thérapeutiquement administrable de celui-ci.

2. Médicament suivant la revendication 1, caractérisé en ce que le principe actif est la diméthylamino-3 méthyl-1(1H, 3H) quinazolinedione-2,4.

3. Médicament suivant la revendication 1, caractérisé en ce que le principe actif est la diéthylamino-3 méthyl-1(1H, 3H) quinazolinedione-2,4.

4. Médicament suivant la revendication 1, caractérisé en ce que le principe actif est la diméthylamino-3 éthyl-1(1H, 3H) quinazolinedione-2,4.

5. Médicament suivant la revendication 1, caractérisé en ce que le principe actif est la chloro-7 diméthylamino-3 éthyl-1(1H, 3H) quinazolinedione-2,4.

6. Médicament suivant la revendication 1, caractérisé en ce que le principe actif est la chloro-7 diméthylamino-3 isopropyl-1(1H, 3H) quinazolinedione-2,4.

**Revendications** (pour l'Etat contractant AT)

1. Procédé de préparation de composés de formule :

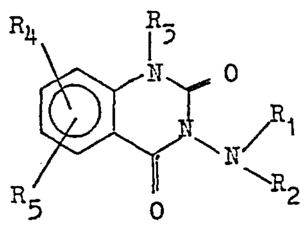

(I)

dans laquelle $R_1$ et $R_2$ représentent chacun, indépendamment l'un de l'autre, un atome d'hydrogène, un groupe alkyle en $C_1$ à $C_4$ ou un groupe acétyle, $R_1$ et $R_2$ n'étant pas simultanément l'hydrogène ; $R_3$ représente un groupe alkyle en $C_1$ à $C_4$ ; et $R_4$ et $R_5$ représentent chacun un atome d'hydrogène ou d'halogène, un groupe alkyle en $C_1$ à $C_4$ ou un groupe alcoxy en $C_1$ à $C_4$ ; et des sels d'addition avec des acides des composés ci-dessus, caractérisé en ce qu'on fait réagir un acide anthranilique de formule

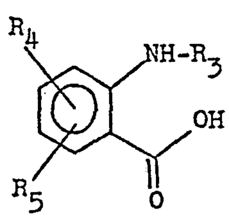

(II)

dans laquelle $R_3$, $R_4$ et $R_5$ ont les définitions précitées, avec du phosgène, obtenant ainsi un anhydride isatoïque de formule

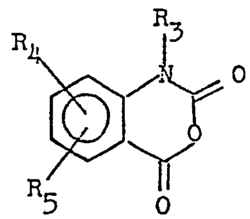

(III)

que l'on fait réagir ensuite sur une hydrazine de formule

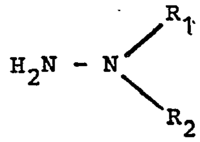

(IV)

dans laquelle $R_1$ et $R_2$ ont les significations précitées, obtenant ainsi un hydrazide anthranilique de formule

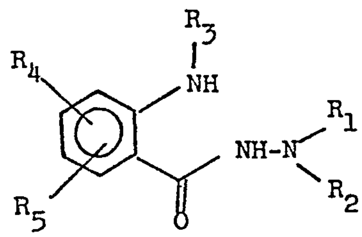

(V)

que l'on cyclise ensuite par réaction avec du phosgène ou du chloroformiate d'éthyle pour obtenir le composé de formule (I).

2. Procédé suivant la revendication 1, caractérisé en ce qu'on fait réagir le phosgène sur l'acide anthranilique de formule (II) en solution acide aqueuse notamment d'acide chlorhydrique, ou en solution dans le dioxane.

3. Procédé suivant la revendication 1 ou 2, caractérisé en ce qu'on effectue la réaction de l'anhydride isatoïque (III) avec l'hydrazine (IV) à température ambiante en suspension dans un alcool ou dans un éther alcoylique de l'éthylène glycol.

4. Procédé suivant l'une quelconque des revendications 1 à 3, caractérisé en ce qu'on effectue la cyclisation de l'hydrazide de formule (V) par le phosgène en présence d'acide chlorhydrique.

5. Procédé suivant l'une quelconque des revendications 1 à 3, caractérisé en ce qu'on effectue la cyclisation de l'hydrazide de formule (V) avec le chloroformiate d'éthyle à la température de reflux du mélange réactionnel.

6. Procédé suivant la revendication 1, caractérisé en ce que, pour obtenir un composé de formule (I) dans lequel $R_1$ et $R_2$ sont tous deux des groupes acétyle, on fait réagir le composé correspondant de formule (I) dans lequel $R_1$ ou $R_2$ est un groupe acétyle avec un agent d'acétylation pour introduire le second groupe acétyle.

7. Procédé suivant la revendication 1, caractérisé en ce qu'on prépare la diméthylamino-3-méthyl-1 (1H, 3H) quinazolinedione-2,4.

8. Procédé suivant la revendication 1, caractérisé en ce qu'on prépare la diéthylamino-3 méthyl-1 (1H, 3H) quinazolinedione-2,4.

9. Procédé suivant la revendication 1, caractérisé en ce qu'on prépare la diméthylamino-3 éthyl-1 (1H, 3H) quinazolinedione-2,4.

10. Procédé suivant la revendication 1, caractérisé en ce qu'on prépare la chloro-7 diméthylamino-3 éthyl-1 (1H, 3H) quinazolinedione-2,4.

11. Procédé suivant la revendication 1, caractérisé en ce qu'on prépare la chloro-7 diméthylamino-3 isopropyl-1 (1H, 3H) quinazolinedione-2,4.

**Claims** (for the Contracting States : DE, NL, SE)

1. Medicament having anxiolytic, diuretic and spasmolytic action usable for the treatment of hypertension, characterized in that it contains, as active ingredient, a compound having the formula :

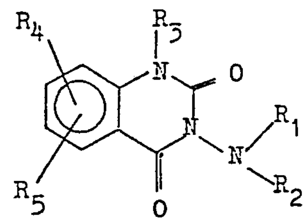

(I)

in which $R_1$ and $R_2$ represent independently from each other a hydrogen atom, a $C_{1-4}$ alkyl group or an acetyl group, $R_1$ and $R_2$ not being simultaneously hydrogen ; $R_3$ represents a $C_{1-4}$ alkyl group ; and $R_4$ and $R_5$ represent independently a hydrogen atom, a halogen atom, a $C_{1-4}$ alkyl group or a $C_{1-4}$ alkoxy group ; or a therapeutically administrable acid addition salt thereof.

2. Medicament according to claim 1, characterized in that the active ingredient is 3-dimethylamino-1-methyl (1H, 3H) quinazoline-2,4-dione.

3. Medicament according to claim 1, characterized in that the active ingredient is 3-diethylamino-1-methyl (1H, 3H) quinazoline-2,4-dione.

4. Medicament according to claim 1, characterized in that the active ingredient is 3-dimethylamino-1-ethyl (1H, 3H) quinazoline-2,4-dione.

5. Medicament according to claim 1, characterized in that the active ingredient is 7-chloro-3-dimethylamino-1-ethyl (1H, 3H) quinazoline-2,4-dione.

6. Medicament according to claim 1, characterized in that the active ingredient is 7-chloro-3-dimethylamino-1-isopropyl (1H, 3H) quinazoline-2,4-dione.

**Claims** (for the Contracting State AT)

1. Process for the preparation of compounds of the formula

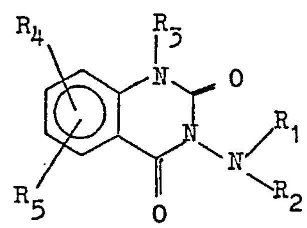

(I)

in which $R_1$ and $R_2$ represent independently from each other a hydrogen atom, a $C_{1-4}$ alkyl group or an acetyl group ; $R_3$ represents a $C_{1-4}$ alkyl group ; and $R_4$ and $R_5$ represent independently a hydrogen atom, a halogen atom, a $C_{1-4}$ alkyl group or a $C_{1-4}$ alkoxy group ; and the acid addition salts of the above compounds, characterized in that an anthranilic acid of the formula

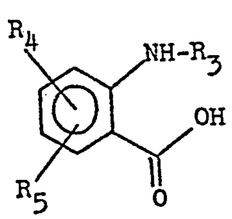

(II)

in which $R_3$, $R_4$ and $R_5$ have the above-defined meanings, is reacted with phosgene, to give an isatoic anhydride having the formula

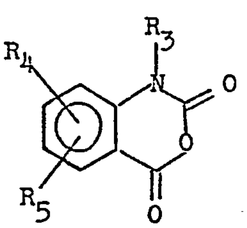

(III)

which is then reacted with a hydrazine having the formula

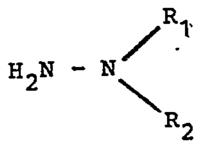

(IV)

in which $R_1$ and $R_2$ have the above-defined meanings, to give an anthranilic hydrazide having the formula

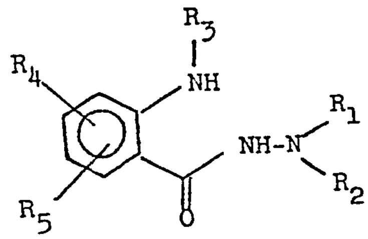

(V)

which is then cyclized by reaction with phosgene or ethyl chloroformate, to give the compound of the formula (I).

2. Process according to claim 1, characterized in that phosgene is reacted with anthranilic acid of the formula (II) in an acid aqueous solution, particularly of hydrochloric acid, or in solution in dioxane.

3. Process according to claim 1 or 2, characterized in that the reaction of isatoic anhydride (III) with hydrazine (IV) is effected at room temperature as a suspension in an alcohol or in an ethylene glycol alkyl ether.

4. Process according to any one of claims 1 to 3, characterized in that the cyclization of hydrazide (V) is effected with phosgene in the presence of hydrochloric acid.

5. Process according to any one of claims 1 to 3, characterized in that the cyclization of hydrazide (V) is effected with ethyl chloroformate at the refluxing temperature of the reaction mixture.

6. Process according to claim 1, characterized in that to obtain a compound of the formula (I) in which $R_1$ and $R_2$ are both acetyl groups, the corresponding compound of the formula (I) in which $R_1$ or $R_2$ is an acetyl group is reacted with an acetylating agent, to introduce the second acetyl group.

7. Process according to claim 1, characterized in that 3-dimethylamino-1-methyl (1H, 3H)-quinazoline-2,4-dione is prepared.

8. Process according to claim 1, characterized in that 3-diethylamino-1-methyl (1H, 3H)-quinazoline-2,4-dione is prepared.

9. Process according to claim 1, characterized in that 3-dimethylamino-1-ethyl (1H, 3H)-quinazoline-2,4-dione is prepared.

10. Process according to claim 1, characterized in that 7-chloro-3-dimethylamino-1-ethyl (1H, 3H) quinazoline-2,4-dione is prepared.

11. Process according to claim 1, characterized in that 7-chloro-3-dimethylamino-1-isopropyl (1H, 3H) quinazoline-2,4-dione is prepared.

**Ansprüche** (für die Vertragsstaaten : DE, NL, SE)

1. Für die Behandlung der Hypertension verwendbares Medikament mit anxiolytischer, diuretischer und spasmolytischer Aktivität, dadurch gekennzeichnet, daß es als Wirkstoff eine Verbindung der Formel

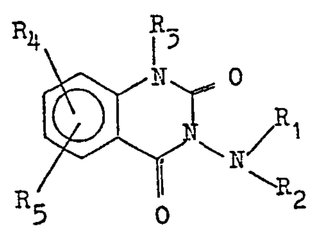

(I)

in der $R_1$ und $R_2$ jeweils unabhängig voneinander ein Wasserstoffatom, ein $C_1$-$C_4$-Alkylrest oder eine Acetylgruppe sind, wobei $R_1$ und $R_2$ nicht gleichzeitig Wasserstoff sind, $R_3$ ein $C_1$-$C_4$-Alkylrest ist und $R_4$ und $R_5$ jeweils ein Wasserstoff- oder Halogenatom, ein $C_1$-$C_4$-Alkylrest oder ein $C_1$-$C_4$-Alkoxyrest sind, oder ein Additionssalz dieser Verbindung mit einer therapeutisch verabreichbaren Säure enthält.

2. Medikament nach Anspruch 1, dadurch gekennzeichnet, daß der Wirkstoff 3-Dimethylamino-1-methyl-(1H, 3H)-2,4-chinazolindion ist.

3. Medikament nach Anspruch 1, dadurch gekennzeichnet, daß der Wirkstoff 3-Diethylamino-1-methyl-(1H, 3H)-2,4-chinazolindion ist.

4. Medikament nach Anspruch 1, dadurch gekennzeichnet, daß der Wirkstoff 3-Dimethylamino-1-ethyl-(1H, 3H)-2,4-chinazolindion ist.

5. Medikament nach Anspruch 1, dadurch gekennzeichnet, daß der Wirkstoff 7-Chlor-3-dimethylamino-1-ethyl-(1H, 3H)-2,4-chinazolindion ist.

6. Medikament nach Anspruch 1, dadurch gekennzeichnet, daß der Wirkstoff 7-Chlor-3-dimethylamino-1-isopropyl-(1H, 3H)-2,4-chinazolindion ist.

**Ansprüche** (für den Vertragsstaat AT)

1. Verfahren zur Herstellung von Verbindungen der Formel

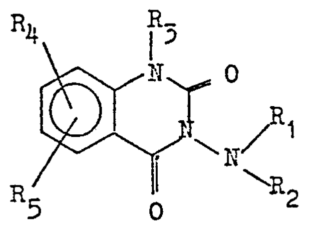

(I)

in der $R_1$ und $R_2$ jeweils unabhängig von einander ein Wasserstoffatom, ein $C_1$-$C_4$-Alkylrest oder eine Acetylgruppe sind, wobei $R_1$ und $R_2$ nicht gleichzeitig Wasserstoff sind, $R_3$ ein $C_1$-$C_4$-Alkylrest ist und $R_4$ und $R_5$ jeweils ein Wasserstoff- oder Halogenatom, ein $C_1$-$C_4$-Alkylrest oder ein $C_1$-$C_4$-Alkoxyrest sind, und der Additionssalze der vorstehend genannten Verbindungen mit Säuren, dadurch gekennzeichnet, daß man eine Anthranilsäure der Formel

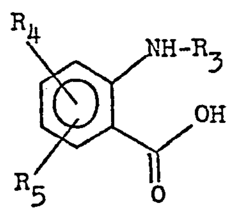

(II)

in der $R_3$, $R_4$ und $R_5$ die vorstehend genannten Bedeutungen haben, mit Phosgen umsetzt, wobei man so ein Isatonsäureanhydrid der Formel

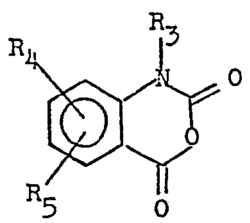

(III)

erhält, das man anschließend mit einem Hydrazin der Formel

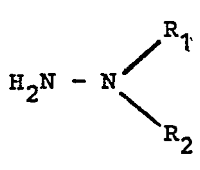

(IV)

in der $R_1$ und $R_2$ die vorstehend genannten Bedeutungen haben, umsetzt und so ein Anthranilhydrazid der Formel

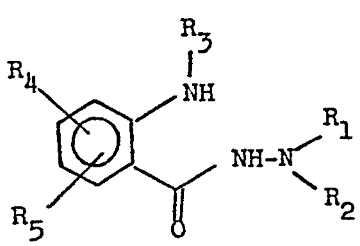

(V)

erhält, das man anschließend durch Umsetzung mit Phosgen oder Ethylchloroformiat cyclisiert, um die Verbindung der Formel (I) zu erhalten.

2. Verfahren nach Anspruch 1, dadurch gekennzeichnet, daß man das Phosgen mit in wäßriger Säure, insbesondere Salzsäure, oder in Dioxan gelöster Anthranilsäure der Formel (II) umsetzt.

3. Verfahren nach Anspruch 1 oder 2, dadurch gekennzeichnet, daß man die Reaktion des Isatonsäureanhydrids (III) mit dem Hydrazin (IV) bei Umgebungstemperatur in Suspension in einem Alkohol oder in einem Alkylether von Ethylenglykol durchführt.

4. Verfahren nach irgendeinem der Ansprüche 1 bis 3, dadurch gekennzeichnet, daß man die Cyclisierung des Hydrazids der Formel (V) mit Phosgen in Gegenwart von Salzsäure durchführt.

5. Verfahren nach irgendeinem der Ansprüche 1 bis 3, dadurch gekennzeichnet, daß man die Cyclisierung des Hydrazids der Formel (V) mit Ethylchloroformiat bei der Rückflußtemperatur des Reaktionsgemisches durchführt.

6. Verfahren nach Anspruch 1, dadurch gekennzeichnet, daß man zum Erhalt einer Verbindung der Formel (I), in der $R_1$ und $R_2$ alle beide Acetylgruppen sind, die entsprechende Verbindung der Formel (I), in der $R_1$ oder $R_2$ eine Acetylgruppe ist, mit einem Acetylierungsmittel umsetzt, um die zweite Acetylgruppe einzuführen.

7. Verfahren nach Anspruch 1, dadurch gekennzeichnet, daß man 3-Dimethylamino-1-methyl-(1H, 3H)-chinazolindion herstellt.

8. Verfahren nach Anspruch 1, dadurch gekennzeichnet, daß man 3-Diethylamino-1-methyl-(1H, 3H)-2,4-chinazolindion herstellt.

9. Verfahren nach Anspruch 1, dadurch gekennzeichnet, daß man 3-Dimethylamino-1-ethyl-(1H, 3H)-2,4-chinazolindion herstellt.

10. Verfahren nach Anspruch 1, dadurch gekennzeichnet, daß man 7-Chlor-3-dimethylamino-1-ethyl-(1H, 3H)-2,4-chinazolindion herstellt.

11. Verfahren nach Anspruch 1, dadurch gekennzeichnet, daß man 7-Chlor-3-dimethylamino-1-isopropyl-(1H, 3H)-2,4-chinazolindion herstellt.